# EUROPEAN PATENT SPECIFICATION

(11) **EP 3 224 378 B1**
(45) Date of publication and mention of the grant of the patent: **17.04.2019**
(21) Application number: 15807612.5
(22) Date of filing: 26.11.2015
(51) Int. Cl.: C12Q 1/6883

(54) **PREDICTION OF NEONATAL ABSTINENCE SYNDROME**
VORHERSAGE DES ABSTINENZSYNDROMS BEI NEUGEBORENEN
PRÉDICTION DU SYNDROME DE SEVRAGE NÉONATAL

(30) Priority: 27.11.2014 GB 201421097
(43) Date of publication of application: 04.10.2017
(73) Proprietor: Bournemouth University, Bournemouth Dorset BH8 8ES (GB)
(72) Inventor: OSSELTON, David, Poole Dorset BH12 5BB (GB); MCLAUGHLIN, Poppy, Poole Dorset BH12 5BB (GB); MACTIER, Helen, Glasgow Strathclyde G31 2ER (GB)
(74) Representative: Gill Jennings & Every LLP
(86) International application number: PCT/EP2015/077786
(87) International publication number: WO 2016/083512

(56) References cited:
- ELISHA M. WACHMAN ET AL: "Association of OPRM1 and COMT Single-Nucleotide Polymorphisms With Hospital Length of Stay and Treatment of Neonatal Abstinence Syndrome", JAMA THE JOURNAL OF THE AMERICAN MEDICAL ASSOCIATION, vol. 309, no. 17, 1 May 2013 (2013-05-01), page 1821, XP055248288, US ISSN: 0098-7484, DOI: 10.1001/jama.2013.3411
- BOGEN D L ET AL: "Pharmacologic evidence to support clinical decision making for peripartum methadone treatment", PSYCHOPHARMACOLOGY, SPRINGER, BERLIN, DE, vol. 225, no. 2, 25 August 2012 (2012-08-25), pages 441-451, XP035158522, ISSN: 1432-2072, DOI: 10.1007/S00213-012-2833-7
- BRITTANY B. DENNIS ET AL: "Impact of ABCB1 and CYP2B6 Genetic Polymorphisms on Methadone Metabolism, Dose and Treatment Response in Patients with Opioid Addiction: A Systematic Review and Meta-Analysis", PLOS ONE, vol. 9, no. 1, 29 January 2014 (2014-01-29), page e86114, XP055248368, DOI: 10.1371/journal.pone.0086114
- ORNA LEVRAN ET AL: "CYP2B6 SNPs are associated with methadone dose required for effective treatment of opioid addiction", ADDICTION BIOLOGY, vol. 18, no. 4, 25 July 2011 (2011-07-25), pages 709-716, XP055248370, GB ISSN: 1355-6215, DOI: 10.1111/j.1369-1600.2011.00349.x
- GABRIELLE L. MCLEMORE ET AL: "Novel pharmacotherapeutic strategies for treatment of opioid-induced neonatal abstinence syndrome", SEMINARS IN FETAL AND NEONATAL MEDICINE, vol. 18, no. 1, 1 February 2013 (2013-02-01), pages 35-41, XP055248383, GB ISSN: 1744-165X, DOI: 10.1016/j.siny.2012.09.002 cited in the application
- Hannah Bunten ET AL: "CYP2B6 and OPRM1 gene variations predict methadone-related deaths : CYP2B6 and OPRM1 gene variations", ADDICTION BIOLOGY, vol. 16, no. 1, 29 November 2010 (2010-11-29), pages 142-144, XP055511559, GB ISSN: 1355-6215, DOI: 10.1111/j.1369-1600.2010.00274.x

## Description

### BACKGROUND

Opioid abuse during pregnancy by way of illicit heroin use or prescribed opioid analgesics can be as high as 21-23% depending upon the demographic (Mcelmore 2013; Minozzi 2013). Prescription-based opioids include codeine, fentanyl, morphine, oxycodone, propoxyphene, buprenorphine and methadone. Although opioid withdrawal in healthy adults is not life-threatening, fetal death in pregnant women and the development neonatal abstinence syndrome (NAS) in the baby is not uncommon. NAS manifests itself in the neonate when the drug supply is interrupted following birth. Symptoms of NAS are non-specific and may involve irritability, high-pitched crying, feeding intolerance, tremors, diarrhea, sickness, respiratory problems and seizures. Opioid substitution therapy (methadone, buprenorphine and morphine) is used to treat drug addicted mothers during pregnancy the main rationale being to encourage prenatal care, prevent complications of illicit opioid use and thus reduce harm to the fetus. Opioid replacement therapy using morphine, methadone and buprenorphine is used for neonates with NAS.

The CYP2B6 protein is a member of the Cytochrome P450 (CYP) family of enzymes which are involved in the metabolism of drugs and other xenobiotics. The CYP genes, notably CYP2B6, are highly polymorphic resulting in a pronounced variability in the expression and enzymatic activities of CYP enzymes which can result in large inter-individual variation in the metabolism of CYP2B6 substrates. The chromosomal location of the CYP2B6 gene is 19q13.2A. Common CYP2B6 polymorphisms are CYP2B6*4 which corresponds to 15631G>T representing a change of guanine to threonine on exon 4 of the CYP2B6 gene (corresponds to 516G>T cDNA), CYP2B6*9 which corresponds to 18053A>G representing a change of adenosine to guanine on exon 5 of the CYP2B6 gene (corresponds to 785G>A cDNA) and the haplotype CYP2B6*6 which incorporates both 15631G>T and 18053A>G (Zanger and Klein 2013); these single nucleotide polymorphisms (SNPs) lead to the amino acid changes Q172H (glutamine to histidine) and K262R (lysine to arginine) in the expressed CYP2B6 protein. Drugs metabolized by CYP2B6 include bupropoion, meperidine, ketamine and methadone.

The inter-individual variability in the development of NAS in neonates and the non-specific nature of NAS makes diagnosis difficult and necessary treatment may be delayed or over-looked. Methods which help counter this deficiency are therefore required.

### References

Bunten H., et al. (2011). Interindividual Variability in the Prevalence of OPRM1 and CYP2B6 GeneVariations May Identify Drug-Susceptible Populations. Journal of Analytical Toxicology, 35: 431-437.
Bunten H., et al.(2010). OPRM1 and CYP2B6 Gene Variants as Risk Factors in Methadone-Related Deaths. Clinical Pharmacology and Therapeutics, 88: 383-389. Mclemore G.L. et al. (2013). Novel Pharmacotherapeutic Startegies for Treatment of Opiod-Induced Neonatal Abstinence Syndrome. Seminars in Fetal and Neonatal Medicine, 18: 35-41.
Minozzi S. et al. (2013). Maintenance agonist treatments for opioid-dependent pregnant women (Review). Cochrane Database of Systematic Reviews, Issue 12, pp. 1-53, Art. No. CD006318. DOI: 10.1002/14651858.CD006318.pub3.
Zanger U.M. and Klein K.(2013). Pharmacogenetics of Cytochrome P450 2B6 (CYP2B6): Advances on Polymorphisms, Mechanisms, and Clinical Relevance. Frontiers in Genetics, 4: 1-12.

### FIGURES

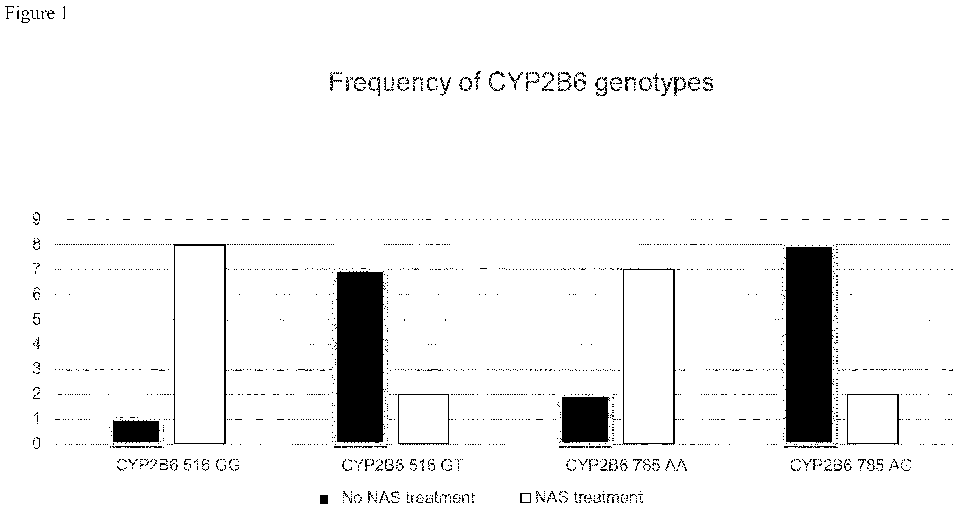

Figure 1 Genotype frequency of neonates in relation to NAS onset

### SUMMARY OF THE INVENTION

It has been found that knowledge of the polynucleotide sequence of the CYP2B6 gene in neonates born to drug addicted women can support a prediction of NAS development. Specifically, the nucleotides present at positions 15631 and 18053 of the CYP2B6 gene can help predict whether the neonate will develop NAS, thus enabling the methods and kits described herein.

### DETAILED DESCRIPTION OF THE INVENTION

The invention describes a method which supports the prediction of the onset of neonatal abstinence syndrome (NAS) in a neonate comprising testing an *ex vivo* biological sample taken from the neonate for the presence or absence of one or more single nucleotide polymorphisms selected from 15631G>T and 18053A>G, where the numbers 15631 and 18053 represents the nucleotide position on the CYP2B6 gene. The mother of the neonate is or has been addicted to drugs, is or has undergone drug addiction therapy, or is taking or has taken drugs; the drugs in question are of the opioid class and is especially methadone with or without morphine and buprenorphine. The opioid class of drugs act by binding to opioid receptors. The method is especially suited to neonates whose mothers are subject to drug addiction therapy during pregnancy; methadone with or without morphine and buprenorphine typify the opioid class of drugs administered to pregnant mothers undergoing drug addiction therapy. The CYP2B6 gene has Gene ID 1555, the SNP 15361G>T has reference number rs3745274, and the SNP 18053A>G has reference number rs2279343 on the National Center for Biotechnology Information (NCBI) database, for which the web addresses are http://www.ncbi.nlm.gov/gene and http://www.ncbi.nlm.gov/SNP. 15631G>T and 18053A>G can also be referred to as rs3745274G>T and rs2279343A>G, respectively. It has been found that the risk of NAS in a neonate is decreased if one or more of the SNPs rs3745274G>T and rs2279343A>G are present i.e. the presence of the homozygous wild type genotype in a neonate at loci 15631 and 18053, namely 15631GG and 18053AA, leads to an increased risk of NAS. In the context of the invention, when referring to the detection of one or more single nucleotide polymorphisms selected from rs3745274G>T and/or rs2279343A>G, the detected SNPs can originate from CYP2B6*4, CYP2B6*9 and the haplotype CYP2B6*6. The * nomenclature is described at The Human Cytochrome P450 (CYP) Allele Nomenclature Database homepage http://www.cypalleles.ki.se. Thus in a preferred embodiment of the method of the invention the presence of one or both of the alleles rs3745274G>T and rs2279343A>G indicates that the neonate is less likely to experience neonatal abstinence syndrome. To prevent the onset of NAS in a neonate who does not possesses one or more of rs3745274G>T and rs2279343A>G it may be necessary to provide a suitable dose of opioid such as methadone, buprenorphine and morphine.

It is thought that the presence of one or more of the SNPs rs3745274G>T and rs2279343A>G at exons 4 and 5 on the CYPB26 gene in the neonate's DNA results in a reduced ability to metabolize opioid drugs such as methadone. This leads to an extension of the systemic residence time of opioid drug making the onset of NAS less likely. Conversely, a negative result i.e. confirmation that the neonatal has a homozygous wild type genotype at nucleotides 15631 and 18053 on exons 4 and 5 on the CYPB26 gene implies that the neonate possesses a normal ability to metabolize a selection of opioid drugs and that the onset of NAS is more likely.

The skilled person would recognise that the absence of the SNPs at loci 15631 and 18053 of the CYP2B6 gene of an individual implies that the individual is homozygous wild type at these positions i.e 15631GG and 18053AA. Hence in *all* the embodiments of the invention it is equally valid to add supplementary information about the individual by identifying the presence of *wild type* alleles at loci 15361 and 18053 of the CYP2B gene. If rs3745274G>T and rs2279343A>G are not identified during *in vitro* sample testing of an individual this would suggest that the individual's genotype is homozygous wild type. It is also standard in the art that detection of the corresponding transcribed mRNA polynucleotides is an alternative to DNA detection. The nucleotides residing at positions 15361 and 18053 of the CYP2B6 gene correspond to positions 516 and 785 of CYPB2B6 mRNA and for all embodiments of the invention detection of the various allelic variants of CYP2B6 mRNA (516G>T and 785A>G) is included.

An embodiment of the invention is a method of predicting the onset of opioid-related neonatal abstinence syndrome (NAS) in a neonatal comprising testing an *ex vivo* biological sample taken from the neonate for the presence or absence of one or more single nucleotide polymorphisms selected from rs3745274G>T and rs2279343A>G which may further comprise the administration to the neonate of an opioid drug which is preferably one or more of methadone, buprenorphine and morphine if the single nucleotide polymorphisms rs3745274G>T and rs2279343A>G are absent. The method is preferably applied to a neonate of a mother who undergoes drug addiction therapy during pregnancy. Although any biological sample is suitable for DNA analysis preferred biological samples are hair or saliva. A saliva sample can be readily obtained from a neonate using a buccal swab.

The single nucleotide polymorphism may be detected, as stated previously is standard in the art, at the nucleic acid (RNA or DNA) or protein level. Several methods, including high throughput methods, are available for detection of SNPs and /or other allelic variations, for example the PCR, restriction fragment length polymorphisms methods and antibody-based methods. DNA from a sample is sequenced (re-sequenced) by any method to identify a SNP or small allelic variation.

A large variety of re-sequencing methods are known in the art, including high-throughput methods. Amplification-based methods are also available to identify allelic variations, such as SNPs, including: PCR, reverse transcriptase PCR (RTPCR), isothermic amplification, nucleic acid sequence based amplification (NASBA), 5' fluorescence nuclease assay (for example TAQMAN assay), molecular beacon assay and rolling circle amplification. Other methods, such as Restriction Fragment Length Polymorphisms RFLP, also may be employed - as is appropriate to identify variant allele(s). Assays may be multiplexed, meaning two or more reactions are conducted simultaneously in the same physical location such as in the same tube or on the same substrate such as a biochip, ensuring the reaction products of the multiplexed reactions can be distinguished. For example, TAQMAN or molecular beacon assays can be multiplexed by use of and by monitoring of accumulation or depletion of two different fluorochromes corresponding to two different sequence-specific probes. In most cases, the appropriate method depends upon personal experience, available equipment and reagents, the need for high throughput and/or multiplexed methods, cost, required accuracy, and the skill levels of the technicians responsible for assay implementation. Design and implementation of these techniques are broadly-known and readily applicable by the skilled person in the art. In the implementation of the methods described herein, an array may be utilized. Arrays are particularly useful for high throughput assays. The array typically comprises one or more reagents, for example, nucleic acid primers and/or probes, for identifying in a nucleic acid sample from a subject the occurrence of an allelic variation corresponding to one or more SNPs. As used herein, the term 'array' refers to reagents facilitating identification of allelic variations in a gene located at two or more identifiable locations. In one embodiment, an array is an apparatus at two or more discrete, identifiable reaction chambers, such as a 96 well dish, in which reactions comprising identified constituents are performed. In an exemplary embodiment, two or more nucleic acid primers or probes are immobilized onto a substrate in a spatially addressable manner so that each individual primer or probe is located at a different and (addressable) identifiable location on the substrate.

Substrates include multi-well plates, ceramic chips and beads. The array can comprise two or more sets of beads, with each bead having an identifiable marker, such as a quantum dot or fluorescent tag, so that the beads are individually identifiable using, for example, a flow cytometer. In one embodiment, in the context of the present disclosure, an array may be a multi-well plate containing two or more well reaction chambers with primers for amplifying DNA to identify SNPs or probes for binding specific sequences. As such, reagents, such as probes and primers may be bound or otherwise deposited onto or into specific locations on an array. Reagents may be in any suitable form, including: in solution, dried, lyophilized or glassified. Useful array technologies include, for example, Affymetrix GeneChip® Array, GenChip® CustomSeq® Resequencing Arrays (commercially available from Affymetrix Inc. of Santa Clara, California). Informatics and/or statistical software or other computer-implemented processes for analyzing array data and/or identifying genetic risk factors from data obtained from a patient sample, are known in the art. Detailed methods to detect rs3745274G>T and rs2279343A>G SNPs and ascribe neonatal genotypes are described in Bunten et al 2011 and Bunten et al 2010.

The invention further describes the use of a kit comprising reagents for detecting the presence or absence of the SNPs rs3745274G>T and rs2279343A>G to inform the administration of a therapeutic drug to a neonate at risk of neonatal abstinence syndrome. The kit reagents comprise a probe which specifically binds to a polynucleotide comprising either rs3745274G>T or rs2279343A>G; it is more preferable that the kit reagents comprises two probes, one which specifically binds to a polynucleotide comprising rs3745274G>T and one which specifically binds to a polynucleotide comprising rs2279343A>G. The use of the kit is preferably applied to a neonate of a mother who undergoes drug addiction therapy during pregnancy. In an embodiment, the kit can kit comprise additional reagents for detecting the presence or absence of the alleles 15631G and 18053A of the CYP2B6 gene (the wild type alleles at loci 15631 and 18053 of the CYP2B6 gene); thus in a further embodiment, the additional kit reagents comprise two probes, one which specifically binds to a polynucleotide comprising 15631G and one which specifically binds to a polynucleotide comprising 18053A. Any of these up to four probes suitable for use in identifying polynucleotides comprising 15631G, 18053A, rs3745274G>T and rs2279343A>G can be attached to a suitable substrate. The substrate for all of the possible kits of the invention is preferably a biochip; a ceramic biochip is especially preferred.

The invention further describes a method of identifying a therapeutic management plan for a neonate at risk of neonatal abstinence syndrome comprising testing an *ex vivo* biological sample taken from the neonate for the presence or absence of one or more single nucleotide polymorphisms selected from rs3745274G>T and rs2279343A>G. The method is preferably applied to a neonate of a mother who undergoes drug addiction therapy during pregnancy. Identifying that a neonate is at a decreased/increased risk of developing NAS enables the clinician to conduct further tests and/or formulate a strategy to deal with the potentially life-threatening condition, by, for example, transferring the infant to a specialist unit. The management plan could comprise the administration or the preparation for administration to a NAS-susceptive neonate of a homozygous wild type at the 15631 and 18053 loci of the CYP2B gene, one or more opioid drug such as methadone, buprenorphine and morphine. If the mother is on opioid therapy post birth, the management plan could be to recommend breast-feeding for homozygous wild type neonates i.e. infants at risk of developing NAS.

The invention also describes an opioid drug for use in a method for the treatment of a neonate at risk of or suffering from neonatal abstinence syndrome characterised in that the genotype of the neonate is homozygous wild type at loci 15631 and 18053 of the CYP2B6 gene. The opioid drug is preferably used to treat a neonate of a mother who undergoes drug addiction therapy during pregnancy. In a preferred embodiment, the opioid drug for use in a method for the treatment of a neonate at risk of or suffering from NAS and who is homozygous wild type at loci 15631 and 18053 of the CYP2B6 gene is one or more of morphine, methadone and buprenorphine.

### Methods and results

### Population

- Identified from a large tertiary maternity hospital in Glasgow
- 21 methadone-maintained, opioid-dependent mother/infant pairs
- 32 control mother/infant pairs
- Non-smoking, DEPCAT 1-3 (affluent): n= 15,smoking, DEPCAT 4-7 (deprived): n= 17
- All infants born ≥ 36 completed weeks' gestation

Buccal swabs were obtained from all mother/infant pairs and the saliva subjected to DNA analysis using the LGC KASP genotyping technique service (http://www.lgcgroup.com/services/genotyping/). All samples were taken within 5 days of delivery,prior to discharge from hospital. NAS was determined by need for pharmacological treatment through retrospective casenote review. Statistical analyses were effected using T-test for the demographics and Fisher's exact test was used to analyse genotype in relation to NAS treatment.

**Table 1 Demographic Results**

| | methadone-exposed n = 21 | controls | | p value |
|---|---|---|---|---|
| | | deprived n = 17 | affluent n = 15 | |
| gestation (days)* | 273 (253 - 287) | 278 (266 - 294) | 275 (245 - 287) | 0.15 |
| birth weight (grams)* | 2746 (2198 - 3400) | 3364 (2512 - 4886) | 3413 (2060 - 4204) | < 0.005 |
| maternal age* | 32 (25 - 42) | 28 (18 - 38) | 33 (27 - 38) | 0.011 |
| Smoking in pregnancy | 100% | 82% | 0% | |

| | | | | |
|---|---|---|---|---|
| * Data represents Median values with range in brackets. | | | | |

**Table 2 Neonates requiring/not requiring treatment for NAS in relation to Genotype**

| | CYP2B6 Gene | | | |
|---|---|---|---|---|
| | Locus 15631 | | Locus 18053 | |
| | GG | GT | AA | AG |
| No NAS treatment | 1 | 7 | 2 | 8 |
| NAS treatment | 8 | 2 | 7 | 2 |
| P value | 0.015 | | 0.023 | |

### Conclusions

Methadone-exposed infants who required treatment for NAS were more likely to be wild type homozygous for CYP2B6 i.e 15631GG and 18053AA genotypes (fast metabolisers) compared to infants who did not require treatment (Table 2). Knowledge of an infant's genotype, and specifically the presence or absence of one or both of the SNPs rs3745274G>T and rs2279343A>G, can help predict the onset of NAS. This enables the implementation of an informed management plan for these infants from birth which may include a requirement of opioid therapy.

## Claims

1. A method of predicting the onset of opioid-related neonatal abstinence syndrome comprising testing an *ex vivo* biological sample taken from a neonate for the presence or absence of single nucleotide polymorphisms selected from rs3745274G>T and/or rs2279343A>G.

2. The method of claim 1 in which the presence of one or both of the single nucleotide polymorphisms rs3745274G>T and rs2279343A>G indicates that the neonate is less likely to experience opioid-related neonatal abstinence syndrome.

3. A method according to any of the preceding claims in which the biological sample is saliva or hair.

4. Use of a kit comprising reagents for detecting the presence or absence of rs3745274G>T and rs2279343A>G to identify a neonate in need of opioid therapy for the treatment of opioid-related neonatal abstinence syndrome, wherein the reagents comprise a probe which specifically binds to a polynucleotide comprising rs3745274G>T and/or a probe which specifically binds to a polynucleotide comprising rs2279343A>G.

5. The use of claim 4 in which the kit reagents comprises two probes, one which specifically binds to a polynucleotide comprising rs3745274G>T and one which specifically binds to a polynucleotide comprising rs2279343A>G.

6. A method of identifying a therapeutic management plan for a neonate at risk of opioid-related neonatal abstinence syndrome comprising testing an *ex vivo* biological sample taken from the neonate for the presence or absence of one or more single nucleotide polymorphisms selected from rs3745274G>T and rs2279343A>G.

7. An opioid drug for use in treating a neonate at risk of or suffering from opioid-related neonatal abstinence syndrome **characterised in that** the genotype of the neonate at the following loci of the CYP2B6 gene is 15631GG and 18053AA.

8. The opioid drug for use according to claim 7 which is one or more of methadone, morphine and buprenorphine.

## Patentansprüche

1. Verfahren zur Vorhersage des Auftretens von Opioid-verbundenem neonatalem Entzugssyndrom, das das Testen einer biologischen *ex-vivo*-Probe, die von einem Neugeborenen entnommen wurde, auf die Gegenwart oder Abwesenheit von Einzelnukleotid-Polymorphismen, die aus rs3745274G>T und/oder rs2279343A>G ausgewählt sind, umfasst.

2. Verfahren nach Anspruch 1, in dem die Gegenwart von einem oder beiden der Einzelnukleotid-Polymorphismen rs3745274G>T und rs2279343A>G anzeigt, dass es für das Neugeborene weniger wahrscheinlich ist, ein Opioid-verbundenes neonatales Entzugssyndrom zu erfahren.

3. Verfahren nach einem der vorstehenden Ansprüche, in dem die biologische Probe Speichel oder Haar ist.

4. Verwendung eines Kits, der Reagenzien für den Nachweis der Gegenwart oder Abwesenheit von rs3745274G>T und rs2279343A>G umfasst, um ein Neugeborenes zu identifizieren, das Opioid-Therapie für die Behandlung von Opioid-verbundenem neonatalem Entzugssyndrom benötigt, wobei die Reagenzien Folgendes umfassen: eine Sonde, die spezifisch zu einem Polynukleotid bindet, das rs3745274G>T umfasst, und/oder eine Sonde, die spezifisch zu einem Polynukleotid bindet, das rs2279343A>G umfasst.

5. Verwendung nach Anspruch 4, in der die Kit-Reagenzien zwei Sonden umfassen, eine, die spezifisch zu einem Polynukleotid bindet, das rs3745274G>T umfasst, und eine, die spezifisch zu einem Polynukleotid bindet, das rs2279343A>G umfasst.

6. Verfahren zur Identifizierung eines therapeutischen Managementplans für ein Neugeborenes, bei dem das Risiko von Opioid-verbundenem neonatalem Entzugssyndrom besteht, das das Testen einer biologischen *ex-vivo*-Probe, die von dem Neugeborenen entnommen wurde, auf die Gegenwart oder Abwesenheit von einem oder mehreren Einzelnukleotid-Polymorphismen, die aus rs3745274G>T und rs2279343A>G ausgewählt sind, umfasst.

7. Opioid-Arzneimittel für die Verwendung bei der Behandlung eines Neugeborenen, bei dem das Risiko von Opioid-verbundenem neonatalem Entzugssyndrom besteht oder das daran leidet, **dadurch gekennzeichnet, dass** der Genotyp des Neugeborenen an den folgenden Loci des CYP2B6-Gens 15631GG und 18053AA ist.

8. Opioid-Arzneimittel für die Verwendung nach Anspruch 7, das eines oder mehrere von Methadon, Morphin und Buprenorphin ist.

## Revendications

1. Méthode de prédiction de la survenue du syndrome de sevrage néonatal associé aux opiacés comprenant tester un échantillon biologique *ex vivo* prélevé d'un nouveau-né pour la présence ou l'absence de polymorphismes d'un seul nucléotide sélectionnés parmi rs3745274G>T et/ou rs2279343A>G.

2. Méthode selon la revendication 1, dans laquelle la présence d'un ou des deux polymorphismes d'un seul nucléotide rs3745274G>T et rs2279343A>G indique que le nouveau-né est moins susceptible de souffrir du syndrome de sevrage néonatal associé aux opiacés.

3. Méthode selon l'une quelconque des revendications précédentes, dans laquelle l'échantillon biologique est de la salive ou des cheveux.

4. Utilisation d'un kit comprenant des réactifs pour détecter la présence ou l'absence de rs3745274G>T et rs2279343A>G afin d'identifier un nouveau-né ayant besoin d'une thérapie à base d'opiacés pour le traitement du syndrome de sevrage néonatal associé aux opiacés, où les réactifs comprennent une sonde qui se lie spécifiquement à un polynucléotide comprenant rs3745274G>T et/ou une sonde qui se lie spécifiquement à un polynucléotide comprenant rs2279343A>G.

5. Utilisation selon la revendication 4, dans laquelle les réactifs du kit comprennent deux sondes, une qui se lie spécifiquement à un polynucléotide comprenant rs3745274G>T et une qui se lie spécifiquement à un polynucléotide comprenant rs2279343A>G.

6. Méthode d'identification d'un plan de prise en charge thérapeutique pour un nouveau-né à risque du syndrome de sevrage néonatal associé aux opiacés comprenant tester un échantillon biologique *ex vivo* prélevé du nouveau-né pour la présence ou l'absence d'un ou de plusieurs polymorphismes d'un seul nucléotide sélectionnés parmi rs3745274G>T et rs2279343A>G.

7. Médicament opiacé à utiliser dans le traitement d'un nouveau-né à risque ou souffrant du syndrome de sevrage néonatal associé aux opiacés, **caractérisé en ce que** le génotype du nouveau-né aux locus suivants du gène CYP2B6 est 15631GG et 18053AA.

8. Médicament opiacé à utiliser selon la revendication 7, qui est un ou plusieurs d'entre la méthadone, la morphine ou la buprénorphine.
